# EUROPÄISCHE PATENTSCHRIFT

(11) **EP 1 509 205 B1**
(45) Veröffentlichungstag und Bekanntmachung des Hinweises auf die Patenterteilung: **20.08.2008**
(21) Anmeldenummer: 03735396.8
(22) Anmeldetag: 15.05.2003
(51) Int. Cl.: A61K 9/14

(54) **PHARMAZEUTISCHE ZUSAMMENSETZUNG MIT VERZÖGERTER WIRKSTOFFFREISETZUNG UND VERFAHREN ZU DEREN ZUBEREITUNG**
PHARMACEUTICAL COMPOSITION HAVING A DELAYED ACTIVE SUBSTANCE RELEASE AND METHOD FOR THE PREPARATION THEREOF
COMPOSITION PHARMACEUTIQUE À LIBÉRATION RETARD DU PRINCIPE ACTIF ET PROCÉDÉ PERMETTANT DE LA PRÉPARER

(30) Priorität: 31.05.2002 DE 10224170
(43) Veröffentlichungstag der Anmeldung: 02.03.2005
(73) Patentinhaber: Desitin Arzneimittel GmbH, D-22335 Hamburg (DE)
(72) Erfinder: FRANKE, Hanshermann, 22889 Tangstedt (DE); LENNARTZ, Peter, 22529 Hamburg (DE); RAIMER, Jörn, 22175 Hamburg (DE)
(74) Vertreter: Gross, Ulrich-Maria
(86) Internationale Anmeldenummer: PCT/EP2003/005115
(87) Internationale Veröffentlichungsnummer: WO 2003/101428

(56) Entgegenhaltungen:
- EP-A- 0 852 140
- EP-A- 1 201 709
- GB-A- 961 813
- US-A- 4 731 122
- SHESKEY P ET AL: "Roll compaction granulation of a controlled-release matrix tablet formulation containing HPMC: Effect of process scale-up on robustness of tablets, tablet stability, and predicted in vivo performance" PHARMACEUTICAL TECHNOLOGY 2000 UNITED STATES, Bd. 24, Nr. 11, 2000, Seiten 30-52, XP002254876 ISSN: 0147-8087 in der Anmeldung erwähnt

## Beschreibung

Die Erfindung betrifft eine pharmazeutische Zusammensetzung mit verzögerter Wirkstofffreisetzung, die insbesondere in Form von einer multiple unit dosage form vorliegt, und ein Verfahren zu ihrer Herstellung.

Bei der Entwicklung von pharmazeutischen Zusammensetzungen mit verzögerter Freisetzung des Wirkstoffs sind sogenannte multiple unit dosage forms (MUD) gegenüber single unit dosage forms (SUD) aufgrund verschiedener Vorteile zu bevorzugen. Die multiple unit dosage forms stellen oral verabreichte Zusammensetzungen dar, die entweder bereits in Form von einer Vielzahl von funktionellen Wirkstoffkompartimenten vorliegen oder nach Einnahme in derartige funktionelle Wirkstoffkompartimente zerfallen. Sie ermöglichen somit unter biopharmazeutischen Aspekten eine optimale Therapie. Beispielsweise ist die Freisetzung des Wirkstoffes aus ihnen weitgehend unabhängig von dem Magenfüllungszustand des Patienten und auch bei verschiedenen Patienten wird eine sehr gleichmäßige Wirkstofffreisetzung erzielt. Schließlich wird das Phänomen des sogenannten "dose dumping" vermieden (vgl. J Butler et al., Pharm. Technol. 1998, Seiten 122 bis 138). Unter "dose dumping" wird das unkontrollierte, schnelle Freisetzen der gesamten oder eines großen Teils der Wirkstoffdosis aus einer-Arzneiform verstanden, die den Wirkstoff eigentlich verzögert und kontrolliert freisetzen soll.

Die Wirkstoffkompartimente einer multiple unit dosage form haben idealerweise in jeder Raumrichtung eine Größe von maximal 2 mm. Nur Kompartimente dieser Größenordnung erzielen den gewünschten optimalen in-vivo Freisetzungsverlauf mit einer nur sehr geringen Beeinflussung durch die Art und Menge der aufgenommenen Nahrung.

Multiple unit dosage forms können z.B. Granulate und Minitabletten mit Teilchengrößen von insbesondere maximal 2 mm in jeder Raumrichtung oder auch Tabletten sein, die nach Einnahme zu Wirkstoff-haltigen Teilchen mit einer Größe von insbesondere maximal 2 mm in jeder Raumrichtung zerfallen.

Den Wirkstoff verzögert freisetzende multiple unit dosage forms werden üblicherweise durch Pelletierung, Mini- oder Mikrotablettierung oder Feuchtgranulation hergestellt. Dabei liegt der Wirkstoff in einer retardierenden Matrix vor oder die Wirkstoffkompartimente sind mit einem retardierenden Film versehen. Für alle diese Verfahrensschritte sind jedoch Lösungsmittel, wie organische Lösungsmittel und Wasser erforderlich. Organische Lösungsmittel sind allerdings ökologisch und toxikologisch bedenklich. Weiter kann die Verwendung von Wasser dazu führen, dass die fertige Arzneiform nicht die gewünschte Stabilität besitzt, weshalb in der Regel aufwendige und unökonomische Trocknungsschritte erforderlich sind. Auch bereits bei der Herstellung kann der Einsatz von Wasser zu Stabilitätsproblemen führen.

Ein bekanntes Verfahren zur Herstellung von Granulaten ohne Lösungsmittel ist die zwanghafte Komprimierung, auch als Kompaktierung bezeichnet, und anschließende Zerkleinerung der verdichteten Feststoffe. Ziel dieser auch als trockene Granulation bezeichneten Variante ist in der Regel die Verbesserung der Fließfähigkeit oder Erhöhung der relativen Dichte der eingesetzten pulverförmigen Materialien. Dabei kann der Wirkstoff alleine oder zusammen mit Hilfsstoffen zwischen zwei Walzen zu einem Strang, auch als Schülpe bezeichnet, verdichtet werden. Dieser Strang wird dann anschließend wieder zu einem Granulat zerkleinert, welches gegenüber der eingesetzten Pulvermischung eine deutlich verbesserte Fließfähigkeit aufweist.

Eine weitere Möglichkeit zur trockenen Herstellung von Granulaten stellt die Brikettierung dar. Bei ihr wird der Wirkstoff allein oder zusammen mit Hilfstoffen zu großen Briketts tablettiert, die dann anschließend wieder auf die gewünschte Größe zerkleinert werden.

Ziel all dieser trockenen Granulierungsverfahren ist die Kompaktierung der eingesetzten Substanzen zur Verbesserung von deren Fließeigenschaften oder auch zur Verbesserung von deren Verpressbarkeit, z.B. bei einer anschließenden Tablettierung.

In der WO 00/08092 wird die Herstellung eines Polyacrylsäure-Granulates durch Verdichtung zwischen zwei Walzen beschrieben. Das hergestellte Granulat wird dann mit Theophyllin als Wirkstoff und weiteren Tablettierhilfsstoffen gemischt und zu Tabletten mit verzögerter Wirkstofffreisetzung verpresst.

Weiter wurden von H. Rey et al. in Drug Development and Industrial Pharmacy 26, 21-26 (2000) Mikrotabletten mit hohem Gehalt an Theophyllin in einer Polymer-Matrix hergestellt. Die Mikrotabletten zeigten eine verzögerte wirkstofffreisetzung und wurden in einer Tablettenpresse aus Granulat hergestellt. Es wurde weiter festgestellt, dass der bei der Tablettierung eingesetzte Komprimierungsdruck keinerlei Einfluss auf das Freisetzungsverhalten des Wirkstoffes hatte.

Paul J. Scheskey et al. beschreiben in Pharm. Technol. Eur. 11/11, 18-35 (1999) die Kompaktierung von Mischungen von Theophyllin mit Hydroxypropylmethylcellulose unter Verwendung von Walzen. Bei der Kompaktierung wurde die Walzentemperatur bei 22°C unter Zuhilfenahme einer zirkulierenden Kühlflüssigkeit gehalten. Die Autoren überprüften den Einfluss verschiedener Parameter des Kompaktierungsverfahrens und stellten dabei fest, dass der eingesetzte Kompaktierungsdruck einen vernachlässigbaren Einfluss auf das Freisetzungsverhalten von Wirkstoff aus entsprechenden Tabletten hatte.

Ein ähnliches Ergebnis erzielten Paul J. Scheskey et al. in Pharmaceutical Technology 18, 132-150 (1994) bei der Untersuchung von verzögert freisetzenden Tabletten mit Niacinamid als Wirkstoff und Methylcellulose oder Hydroxypropylmethylcellulose. Das zur Herstellung der Tabletten eingesetzte Granulat wurde durch Kompaktierung mittels Walzen bei unterschiedlichen Drücken hergestellt. Im Ergebnis konnte zwar ein besseres Fließverhalten erzielt werden, es war jedoch keine Abhängigkeit zwischen eingesetztem Walzendruck und Geschwindigkeit der Freisetzung des Wirkstoffes erkennbar.

Weiter wurden von P. Scheskey et al. in Pharmaceutical Technology 24, 30-52 (2000) die Verfahrensparameter bei der trockenen Granulierung von Theophyllin-Formulierungen mit verzögerter Wirkstofffreisetzung mit dem Ziel untersucht, diese von Laboratoriumsmaßstäben auf industrielle Maßstäbe zu übertragen. Es wurden dabei Kompaktierungskräfte pro linearer Längeneinheit der Walzenbreite von 2,8, 3,1 und 3,4 t/inch eingesetzt, was in SI-Einheiten 10,8 kN/cm, 12,0 kN/cm bzw. 13,2 kN/cm entspricht.

Insgesamt zeigen die vorstehenden Veröffentlichungen, dass die beim Kompaktiervorgang durch die Walzen ausgeübte Kraft einen vernachlässigbaren oder überhaupt keinen Einfluss auf das Freisetzungsprofil von verzögert freisetzenden Arzneiformen hat. In allen Fällen wurde die Kompaktierung zur Beeinflussung von physikalischen Feststoffparametern, nicht aber zur Erzielung einer kontrollierten Freisetzung verwendet.

Allenfalls ist im Falle der Walzenkompaktierung einer Mischung von niedrigsubstituierter Hydroxypropylmethylcellulose und Acetaminophen festgestellt worden, dass bei Erhöhung des Kompaktierdruckes sich die Geschwindigkeit der Freisetzung von Acetaminophen sogar erhöhte (vgl. Y. Kawashima et al. in Chem. Pharm. Bull. 41, 1827-1831 (1993)).

Es besteht somit Bedarf an einer pharmazeutischen Zusammensetzung mit verzögerter Wirkstofffreisetzung und einem Verfahren zu deren Herstellung, bei dem eine Granulierung ohne Zuhilfenahme von organischen Lösungsmitteln oder Wasser erfolgt und welches die Herstellung von Zusammensetzungen insbesondere in Form von multiple unit dosage forms mit ausgezeichnetem Retardeffekt trotz nur geringer Mengen an retardierendem Polymer gestattet.

Diese Aufgabe wird durch das Verfahren gemäß einem der Ansprüche 1 bis 15 und die pharmazeutischen Zusammensetzungen gemäß den Ansprüchen 16 bis 19 gelöst.

Überraschenderweise hat es sich gezeigt, dass durch Auswahl einer bestimmten Kompaktierkraft und Einstellung einer bestimmten Temperatur bei den zum Kompaktieren verwendeten Walzen pharmazeutische Zusammensetzungen gerade in Form von multiple unit dosage forms erhalten werden können, die eine verzögerte Wirkstofffreisetzung haben.

Das erfindungsgemäße Verfahren zur Herstellung einer pharmazeutischen Zusammensetzung mit verzögerter Wirkstofffreisetzung zeichnet sich dadurch aus, dass
(a) eine Mischung bereitgestellt wird, die Wirkstoff und verzögerte Wirkstofffreisetzung bewirkendes Polymer enthält,
(b) die Mischung verdichtet wird, indem sie zwischen zwei Walzen hindurchgeführt wird, die eine Temperatur von mehr als 40°C aufweisen und die eine Kraft im Bereich von mehr als 15 und bis zu 40 kN/cm Walzenbreite auf die Mischung ausüben,
(c) die verdichtete Mischung auf die gewünschte Teilchengröße zerkleinert wird und
(d) ggf. die verdichtete und zerkleinerte Mischung weiterverarbeitet wird.

In Stufe (a) wird eine Mischung von Wirkstoff und Polymer bereitgestellt, wobei das Polymer eine verzögerte Wirkstofffreisetzung bewirkt. Als Wirkstoffe kommen eine ganze Reihe von Substanzen in Frage und insbesondere ist der Wirkstoff aus einer der folgenden Gruppen ausgewählt:
Alpha- oder Beta-Adrenergika,
Alpha- oder Beta-Adrenolytika.
Analgetika,
Antirheumatika,
Antiarthritika,
Anticholinergika,
Antikonvulsiva,
Antidepressiva,
Antiparkinsonmittel,
Antipsychotika,
Anxiolytika,
Dopaminrezeptoragonisten,
Migränemittel,
Neuroleptika,
Neuroprotektiva,
Nootropika,
nicht-steroidale Antirheumatika,
Sedativa und
Hypnotika.

Bevorzugte Vertreter für die obigen Wirkstoffgruppen sind:
Für Antikonvulsiva insbesondere
   10-Hydroxycarbamazepin
   3-Methyl-5-phenylhydantoin
   4-Amino-3-hydroxybuttersäure
   5-Methyl-5-(3-phenanthryl)hydantoin
   Acetazolamid
   Acetylpheneturid
   Albution
   Aloxidon
   Aminoglutethimid
   Aminopentamid
   Atrolactamid
   Beclemid
   Benzodiazepin
   Brom sowie Bromide
   Buramat
   Carbamazepin
   Clobazam
   Clomethiazol
   Clonazepam
   Decimemid
   Dilantin
   Dimethadion
   Diphenylan
   Diphenylhydantoin
   Doxenitoin
   Eterobarb
   Ethadion
   Ethoin
   Ethoxsuximid
   Famotidin
   Felbamat
   Fluoreson
   Fosphenytoin
   Gabapentin,
   Hakoseride
   L-5-Hydroxytryptophan und davon abgeleitete Salze und Komplexverbindungen und Mischungen von diesen,
   Lamotrigin
   Levetiracetam
   Magnesiumsulfat
   Mephenytoin
   Mephobarbital
   Metharbital
   Methetoin
   Methsuximid
   Narcobarbital
   Natrium Divalproex
   Nimetazepam
   Nitrezepam
   Oxcarbazepin
   Paramethadion
   Phenacemid
   Phenetharbital
   Pheneturid
   Phenobarbital
   Phensuximid
   Phenylmethylbarbitursaure
   Phenytoin Natrium
   Phethenylat Natrium
   Pjenytoin
   Pregabalin
   Primidon
   Progabid
   Reboxetin
   Remacemid
   Rufinamid
   Suclofenid
   Sultiam
   Tetrantoin
   Talampanel
   Tiagabin
   Topiramat
   Trimethadion
   Valproat Natrium
   Valproinsäure
   Valpromid
   Vigabatrin
   Zonisamid
Für Antiparkinsonmittel insbesondere:
   Amantadin
   Benserazid
   Bietanautin
   Biperiden
   Budipin
   Cabergolin
   Carbidopa
   Deprenyl
   Dexetimid
   Diethazin
   Droxidopa
   Entacapon
   Ethopropazin
   Ethylbenzhydramin
   Lazabemid
   Levodopa
   Memantin
   Mofegilin
   Piroheptin
   Pramipexol
   Pridinol
   Prodipin
   Ropinirol
   Selegilin
   Talipexol
   Tergurid
   Tiaprid
   Tigloidin
   Tolcapon
   Trihexyphenidyl Hydrochlorid
Für Antipsychotika, Neuroleptika und Antidepressiva insbesondere:
   Acetophenazin
   Alizaprid
   Amantadin
   Amisulprid
   Aripiprazol
   Befloxaton
   Benperidol
   Benserazid
   Benzquinamid
   Bietanautin
   Biperiden
   Bromocriptin
   Bromperidol
   Budipin
   Buramat
   Butaperazin
   Butyrophenon
   Cabergolin
   Carbidopa
   Carbipromin
   Carphenazin
   Carpipramin
   Chlorproethazin
   Chlorpromazin
   Chlorprothixen
   Clocapramin
   Clomacran
   Clopenthixol
   Clospirazin
   Clothiapin
   Clozapin
   Cyamemazin
   Dexetimid
   Diethazin
   Dixyrazin
   Droperidol
   Entacapon
   Ethopropazin
   Ethylbenzhydramin
   Fluanison
   Flupentixol
   Fluphenazin
   Fluspirilen
   Haloperidol
   Iloperidone
   Imiclopazin
   Lazabemid
   Levodopa
   Lisurid
   Melperon
   Memantin
   Mepazin
   Mesoridazin
   Methoxypromazin
   Metofenazat
   Mofegilin
   Molindon
   Moperon
   Mosapramin
   Nemonaprid
   Olanzapin
   Opipramol
   Oxaflumazin
   Penfluridol
   Perazin
   Pergolid
   Pericyazin
   Perimethazin
   Perphenazin
   Phenothiazin
   Pimozid
   Pipamperon
   Piperacetazin
   Piroheptin
   Pramipexol
   Pridinol
   Prochlorperazin
   Prodipin
   Promazin
   Prothipendyl
   Quetiapin
   Remoxiprid
   Retigabin
   Risperidon
   Ropinirol
   Rotigentin
   Selegilin
   Sertindol
   Spiperon
   Sulforidazin
   Sulprid
   Sultoprid
   Talipexol
   Tergurid
   Tetrabenazin
   Thiopropazat
   Thioproperazin
   Thioridazin
   Thiothixen
   Thioxanthen
   Timiperon
   Tolcapon
   Trifluoperazin
   Trifluperidol
   Triflupromazin
   Trihexyphenidyl Hydrochlorid
   Ziprasidon
   Zotepin
Für Dopaminrezeptoragonisten insbesondere:
   Bromocripitin
   Fenoldopam
   Lisurid
   Naxagolid
   Pergolid
Für Neuroprotektiva insbesondere:
   Dizocilpin
   β-Alaninderivate
   Xaliproden

Auch Kombinationen von Wirkstoffen können verwendet werden. Es ist besonders bevorzugt, dass es sich bei dem Wirkstoff um ein Antikonvulsivum, ein Antidepressivum, ein Antiparkinsonmittel, ein Antipsychotikum, ein Anxiolytikum oder einen Dopaminrezeptoragonisten handelt.

Ganz besonders bevorzugt ist als Wirkstoff Oxcarbazepin, Valpro insäure oder deren Salze, Sultiam, Carbamazepin, Lamotrigin oder Levetiracetam.

Als Polymer kommen üblicherweise zur verzögerten Wirkstofffreisetzung bei pharmazeutischen Zusammensetzungen verwendete Polymere in Frage. In erster Linie sind hierbei Polymere oder Copolymere von Acrylsäure oder Acrylsäurederivaten, oder von Methacrylsäure und Methacrylsäurederivaten, Cellulose-Polymere, Wachse oder Fette zu nennen. Weitere geeignete Substanzen sind Polyvinylpyrrolidon (PVP), PVP-Derivate, Polyethylenglykol (PEG), PEG-Derivate, Stärke, Stärkederivate, Polyvinylchlorid, Polyethylen, Polyvinylacetat, Polyvinylalkohol, Cellulosen, wie Ethylcellulose, Methylcellulose, Hydroxypropylmethylcellulose (HPMC), HPMC-Derivate, Hydroxypropylcellulose, Celluloseacetat, Etylen-Vinylacetat-Copolymer, oder Polyvinylacetat-Crotonsäure-Copolymer.

Besonders bevorzugt wird als Polymer ein Acrylsäure- oder Methacrylsäure-Polymer, ein Acrylsäure- oder Methacrylsäure-Copolymer oder ein Salz von diesen verwendet. Auch Mischungen verschiedener Substanzen können als Polymer verwendet werden.

Weiter können dem Wirkstoff und dem Polymer auch noch Hilfsstoffe aus den Gruppen Farbstoffe, Fließregulierungsmittel, Schmiermittel, Trockenbindemittel, Zerfallshilfsmittel und Stabilisatoren zugesetzt werden.

Zur Herstellung der Mischung werden übliche Mischer oder Mühlen verwendet.

Es ist bevorzugt, dass die Mischung 5 bis 90 und insbesondere 70 bis 85 Gew.-% Wirkstoff enthält. Die Menge an Polymer in der Mischung beträgt vorzugsweise 2 bis 50 und insbesondere 5 bis 30 Gew.-% Polymer.

In Stufe (b) erfolgt dann die Verdichtung der Mischung. Dabei wird insbesondere so vorgegangen, dass die Mischung durch geeignete Fördermittel, wie z.B. eine Förderschnecke, zu den sich gegenläufig drehenden zwei Walzen transportiert und zwischen diesen zwei Walzen hindurchgeführt wird. Die Walzen weisen eine Temperatur von mehr als 40°C auf und zu diesem Zwecke sind sie üblicherweise mit einer Thermostatisiereinrichung versehen. Es ist bevorzugt, dass die Temperatur der Walzen 100°C nicht übersteigt. Besonders bevorzugt ist die Temperatur der Walzen im Bereich von 70 bis 90°C.

Weiter ist es wichtig, dass die Walzen eine Kraft auf die Mischung ausüben, die im Bereich von 15 und bis zu 40 kN/cm Walzenbreite beträgt. Die Einstellung dieser Kraft erfolgt in der Regel durch Messung der Verformung am Maschinenrahmen über Dehnungsmeßstreifen oder durch Messung von Drücken im Hydraulikbereich sowie Kalibrierung der Meßeinrichtung über Kraftmeßzellen, welche zwischen die Walzen montiert werden. Die Kraft wird dann über SPS gesteuert. In diesem Zusammenhang bedeutet Walzenbreite die Länge der direkten Verbindungslinie zwischen den Walzenrändern. Mit dieser erfindungsgemäß benutzten Definition entfallen Probleme zur Bestimmung der Walzenbreite, wie sie z.B. bei Walzen mit unregelmäßigen Oberflächen auftreten könnten.

Es hat sich herausgestellt, dass besonders bevorzugt die Walzen eine Kraft im Bereich von 18 bis 23 kN/cm Walzenbreite auf die Mischung ausüben.

Übliche Walzenkompaktoren, die eine Einstellung der auf die zu kompaktierende Mischung ausgeübten Kraft im erfindungsgemäßen Bereich und eine Erwärmung der Walzen auf die erforderliche Temperaturen ermöglichen, sind für das erfindungsgemäße Verfahren geeignet. Geeignet sind beispielsweise 3-W-Polygran-Maschinen von Gerteis Maschinen + Processengineering AG, Jonä, Schweiz.

Infolge der Verdichtung der Mischung durch die zwei Walzen nimmt die Mischung üblicherweise die Form eines Stranges an, der auch als Schülpe bezeichnet wird.

In Stufe (c) wird die verdichtete Mischung auf die gewünschte Teilchengröße zerkleinert, wobei Teilchengrößen von 50 bis 1000 µm in jeder Raumrichtung bevorzugt sind. Die Zerkleinerung auf die gewünschte Teilchengröße erfolgt mit üblichen Mitteln, wie Sieben, Mahlen oder Brechen.

Die nach dieser Stufe vorliegende verdichtete und zerkleinerte Mischung stellt bereits eines multiple unit dosage form, d.h. ein Granulat dar, welches aus funktionellen Wirkstoffkompartimenten besteht.

In Stufe (d) kann die verdichtete und zerkleinerte Mischung dann ggf. noch weiter verarbeitet werden. Üblicherweise wird die Mischung hierzu zunächst klassiert, was z.B. mit Hilfe eines Rüttelsiebes erfolgen kann.

Das klassierte Kompaktat kann dann z.B. in Kapseln oder Minipacks, d.h. kleinen Beutelchen (Sachets) abgepackt werden.

Es ist ebenfalls möglich, dass das klassierte Kompaktat direkt zu Tabletten zusammen mit üblichen Hilfsstoffen verpresst wird.

Weiter ist es ebenfalls möglich, dass das klassierte Kompaktat einer feuchten Granulierung unterworfen wird und das erhaltene Granulat dann zu Tabletten verpresst wird.

Die erhaltenen Tabletten weisen die vorteilhafte Eigenschaften auf, dass sie bei Kontakt mit wässrigen Medien zu Teilchen mit einer Größe von < 2 mm in jeder Raumrichtung zerfallen und somit stellen sie ebenso wie das klassierte Kompaktat eine multiple unit dosage form dar.

Bevorzugt sind erfindungsgemäß solche multiple unit dosage forms, die (a) mehr als 25 Gew.-% an wirkstoffhaltigen Teilchen mit einer Größe von < 2 mm in jeder Raumrichtung enthalten oder (b) die in Gegenwart von wässrigen Medien zu mehr als 25 Gew.-% zu wirkstoffhaltigen Teilchen mit einer Größe von < 2 mm in jeder Raumrichtung zerfallen.

Der Vorteil derartig kleiner Teilchen ist, dass sie im Gegensatz zu monolitischen Darreichungsformen, wie konventionellen Tabletten, den Pylorus ungehindert passieren können.

Bei der Herstellung von erfindungsgemäßen Tabletten können dem Kompaktat auch übliche Zusatzstoffe zugesetzt werden, wie Schmiermittel, Fließregulierungsmittel, Zerfallshilfsmittel, Farbstoffe, Weichmacher, Trennmittel oder Bindemittel. Besonders bevorzugt sind hier mikrokristalline Cellulose, Magnesiumstearat, Aerosil R 972, Natriumcarboxymethylstärke und Triethylcitrat.

Bei der Granulation der verdichteten und zerkleinerten Mischung kann auch wässrige Granulaten erfolgen, da der Wirkstoff durch die Vorbehandlung mit dem Polymer geschützt ist. Die fertige pharmazeutische Zusammensetzung kann schließlich auch noch mit einem schnell zerfallenden Film versehen werden.

Eine Untersuchung der verdichteten und zerkleinerten Mischung zeigt Partikel, in denen das Polymer eine kohärente Matrix ausbildet, die zur verzögerten Freisetzung des Wirkstoffes führt. REM-Aufnahmen zeigen demgemäß Partikel mit homogener Struktur, und es sind keine Partikel des Ausgangsmaterials mehr erkennbar. Es wird angenommen, dass durch die in Stufe (b) erfolgte hohe Verdichtung der Mischung und die kurzzeitig über die walzen eingebrachte erhöhte Temperatur es zu einem Fließen des Polymers um die Wirkstoffpartikel herum kommt, ohne dass das Polymer einen flüssigen Aggregatzustand einnimmt.

Es ist jedenfalls feststellbar, dass auch ohne den Einsatz von organischen Lösungsmitteln und Wasser bei dem erfindungsgemäßen Verfahren pharmazeutische Zusammensetzungen erhalten werden, aus denen der Wirkstoff verzögert freigesetzt wird, obwohl nach dem Stand der Technik die eingesetzte Kompaktierung lediglich zur Fließverbesserung oder Dichteerhöhung des Kompakats dient.

Es zeigte sich, dass die erfindungsgemäß erhaltenen Zusammensetzungen über eine Zeitraum von bis zu 8 Stunden den Wirkstoff freisetzten. Eine unter gleichen Bedingungen untersuchte konventionell erhaltenene Zusammensetzung mit schneller Freisetzung gab den Wirkstoff hingegen bereits innerhalb einer Stunde ab.

Die Erfindung betrifft daher schließlich auch eine pharmazeutische Zusammensetzung, die durch das erfindungsgemäße Verfahren erhältlich ist.

Bevorzugt liegt die erfindungsgemäße Zusammensetzung in Form von Kapseln, Beuteln, Styli, Tabletten oder Minitabletten vor.

Besonders bevorzugt ist eine pharmazeutische Zusammensetzung, die (i) mehr als 25 Gew.-% wirkstoffhaltige Teilchen mit einer Größe von < 2mm in jeder Raumrichtung enthält oder (ii) die bei Kontakt mit wäßrigen Medien zu mehr als 25 Gew.-% zu wirkstoffhaltigen Teilchen mit einer Größe von < 2mm in jeder Raumrichtung zerfällt.

Weiter kann auch eine Mischung von verschiedenen Kompaktaten des gleiche Wirkstoffes eingesetzt werden, um auf diese Weise zu einer erfindungsgemäßen Zusammensetzung zu gelangen, die ein bestimmtes Freisetzungsprofil für den Wirkstoff hat.

Die Erfindung wird im Folgenden anhand von Beispielen näher erläutert.

### Beispiele

### Beispiel 1 (Retardkompaktat)

### Rezeptur:

| | |
|---|---|
| Oxcarbazepin | 30 kg |
| Ammoniummethacrylat-Copolymer (Eudargit RSPO) | 9 kg |

Das Oxcarbazepin wird mit dem Ammoniummethacrylat-Copolymer in einem Schnellmischer (Diosna P 100) 5 Min. lang gemischt. Die Mischung wurde dann mittels eines Kompaktors, nämlich 3-W-Polygran, Gerteis Maschinen + Processengineering AG, Jona, Schweiz, (Walzenbreite 10 cm und Walzengeschwindigkeit 7 Umdrehungen pro Minute) mit einer Kraft von mehr als 15 und bis zu 40 kN/cm Walzenbreite verdichtet, nachdem die Walzen thermostatgesteuert auf 80°C erhitzt wurden. Die entstandene Schülpe wurde mittels Zwangssiebung (1 mm Siebeinsatz) zerkleinert, und das erhaltene Kompaktat wurde über ein Rüttelsieb (Engelmann Siebrinne mit 0,25 mm Siebeinsatz) klassiert. Das klassierte Kompaktat wurde dann auf einer Kapselfüllmmaschine in Hartgelatinekapseln der Größen 3, 2, 1 und 0 abgefüllt. Es entstanden dadurch Dosierungseinheiten von 150 bis 300 mg Wirkstoff pro Einzeldosis.

Weiter wurde das klassierte Kompaktat auch auf einer Beutelmaschine in kleine Beutelchen, auch als Minipacks oder Sachets bezeichnet, abgepackt. Es entstanden dadurch Dosierungseinheiten von 50 bis 2400 mg pro Einzeldosis.

### Beispiel 2 (Retardgranulat)

### Rezeptur:

| | |
|---|---|
| Oxcarbazepin | 30 kg |
| Ammoniummethacrylat-Copolymer (Eudragit RSPO) (1.Teil) | 9 kg |
| 30 %-ige Ammoniummethacrylat-Copolymer-Dispersion in Wasser (Eudragit RS 30 D) | 2,5 kg |
| Ammoniummethacrylat-Copolymer (Eudargit RSPO) (2. Teil) | 2,5 kg |
| Talkum | 0,9 kg |
| Triethylcitrat | 0,2 kg |

Das Oxcarbazepin wurde zunächst mit den ersten Teil des Ammoniummethacrylat-Copolymers in einem Schnellmischer 5 Min. lang gemischt. Die Mischung wurde dann auf einen Kompaktor bei 20 kN/cm Walzenbreite kompaktiert, nachdem die Walzen auf 80°C erhitzt wurden. Die entstandene Schülpe wurde mittels Zwangssiebung zerkleinert und das entstandene Kompaktat wurde über ein Rüttelsieb klassiert. Danach wurde das klassierte Kompaktat in einem Wirbelschichtgranulator (Glatt WSG 60) mit dem zweiten Teil des Ammoniummethacrylat-Copolymers gemischt und mit Hilfe der wässrigen Ammoniummethacrylat-Copolymer-Dispersion unter Zusatz von Triethylcitrat und Talkum granuliert.

Das erhaltene Granulat wurde auf einer Kapselfüllmaschine in Hartgelatinekapseln der Größen 3, 2, 1 und 0 abgefüllt. Es entstanden dadurch Dosierungseinheiten von 150 bis 300 mg Wirkstoff pro Einzeldosis.

Das klassierte Kompaktat wurde weiter auf einer Beutelmaschine in Beutelchen abgepackt und es entstanden dadurch Dosierungen von 500 bis 2400 mg pro Einzeldosis.

### Beispiel 3 (Retardtablette aus Kompaktat)

### Rezeptur:

| | |
|---|---|
| Oxcarbazepin | 30 kg |
| Ammoniummethacrylat-Copolymer | 9 kg |
| Natrium Carboxymethylstärke | 0,2 kg |
| Magnesiumstearat | 0,5 kg |
| Mikrokristalline Cellulose | 10,3 kg |

Das Oxcarbazepin wurde mit dem Ammoniummethacrylat-Copolymer in einem Schnellmischer 5 Min. lang gemischt. Die erhaltene Mischung wurde dann auf einem Walzen-Kompaktor bei 20 kN/cm kompaktiert, nachdem die Walzen auf 80°C erhitzt wurden. Die entstandene Schülpe wurde mittels Zwangssiebung zerkleinert und das erhaltene Kompaktat wurde über ein Rüttelsieb klassiert. Das Kompaktat wurde dann mit der Natrium Carboxymethylstärke, dem Magnesiumstearat und der mikrokristallinnen Cellulose gemischt und zu Tabletten verpresst, wobei Dosierungseinheiten zwischen 150 und 600 mg entstanden.

### Beispiel 4 (Retardtablette aus Granulat)

### Rezeptur:

| | |
|---|---|
| Oxcarbazepin | 30 kg |
| Ammoniummethacrylat-Copolymer (1. Teil) | 9 kg |
| 30 %-ige Ammoniummethacrylat-Copolymer-Dispersion | 2,5 kg |
| Ammoniummethacrylat-Copolymer (2. Teil) | 2,5 kg |
| Talkum | 0,9 kg |
| Triethylcitrat | 0,2 kg |
| Natrium Carboxymethylstärke | 0,2 kg |
| Magnesiumstearat | 0,5 kg |
| Mikrokristalline Cellulose | 5,8 kg |

Das Oxcarbacepin wurde mit dem ersten Teil Ammoniummethacrylat-Copolymer in einem Schnellmischer 5 Min. lang gemischt. Die Mischung wurde dann auf einem Walzen-Kompaktor bei 20 kN/cm Walzenbreite kompaktiert, nachdem die Walzen auf 80°C erhitzt wurden. Die entstandene Schülpe wurde mittels Zwangssiebung zerkleinert und das erhaltene Kompaktat wurde über ein Rüttelsieb klassiert. Dass klassierte Kompaktat wurde dann in einem Wirbelschichtgranulator mit dem zweiten Teil des Ammoniummethacrylat-Copolymers gemischt und mit der wässrigen Polymer-Dispersion unter Zusatz von Triethylcitrat und Talkum granuliert.

Das Granulat wurde dann mit Natrium Carboxymethylstärke, Magnesiumstearat und mikrokristalliner Cellulose gemischt und zu Tabletten verpresst, wobei Dosierungseinheiten zwischen 150 und 600 mg entstanden.

## Patentansprüche

1. Verfahren zur Herstellung einer pharmazeutischen Zusammensetzung mit verzögerter Wirkstofffreisetzung, bei dem
(a) eine Mischung bereitgestellt wird, die Wirkstoff und verzögerte Wirkstofffreisetzung bewirkendes Polymer enthält,
(b) die Mischung verdichtet wird, indem sie zwischen zwei Walzen hindurchgeführt wird, die eine Temperatur von mehr als 40°C aufweisen und die eine Kraft im Bereich von mehr als 15 und bis zu 40 kN/cm Walzenbreite auf die Mischung ausüben,
(c) die verdichtete Mischung auf die gewünschte Teilchengröße zerkleinert wird und
(d) ggf. die verdichtete und zerkleinerte Mischung weiter verarbeitet wird.

2. Verfahren nach Anspruch 1, bei dem die Walzen eine Temperatur von bis zu 100°C aufweisen.

3. Verfahren nach Anspruch 1 oder 2, bei dem die Walzen eine Temperatur im Bereich von 70 bis 90 °C aufweisen.

4. Verfahren nach einem der Ansprüche 1 bis 3, bei dem die Walzen eine Kraft im Bereich von 18 bis 23 kN/cm Walzenbreite auf die Mischung ausüben.

5. Verfahren nach einem der Ansprüche 1 bis 4, bei dem die Mischung 5 bis 90 Gew.-% Wirkstoff enthält.

6. Verfahren nach einem der Ansprüche 1 bis 5, bei dem die Mischung 2 bis 50 Gew.-% Polymer enthält.

7. Verfahren nach einem der Ansprüche 1 bis 6, bei dem der Wirkstoff aus einer der folgenden Gruppen ausgewählt ist
Alpha- oder Beta-Adrenergika,
Alpha- oder Beta-Adrenolytika.
Analgetika,
Antirheumatika,
Antiarthritika,
Anticholinergika,
Antikonvulsiva,
Antidepressiva,
Antiparkinsonmittel,
Antipsychotika,
Anxiolytika,
Dopaminrezeptoragonisten,
Migränemittel,
Neuroleptika,
Neuroprotektiva,
Nootropika,
nicht-steroidale Antirheumatika,
Sedativa und
Hypnotika.

8. Verfahren nach Anspruch 7, bei dem der Wirkstoff ein Antikonvulsivum, ein Antidepressivum, ein Antiparkinsonmittel, ein Antipsychotikum, ein Anxiolytikum oder ein Dopaminrezeptoragonist ist.

9. Verfahren nach Anspruch 8, bei dem der Wirkstoff Oxcarbazepin, Valproinsäure oder ein Salz davon, Sultiam, Carbamazepin, Lamotrigin oder Levetiracetam ist.

10. Verfahren nach einem der Ansprüche 1 bis 9, bei dem das Polymer ein Acrylsäure-Polymer oder Acrylsäure-Copolymer oder ein Salz von diesen ist.

11. Verfahren nach einem der Ansprüche 1 bis 10, bei dem die Mischung in Stufe (a) zusätzlich mindestens eine Substanz aus der Gruppe Farbstoff, Fließregulierungsmittel, Schmiermittel, Trockenbindemittel, Zerfallshilfsmittel und Stabilisator enthält.

12. Verfahren nach einem der Ansprüche 1 bis 11, bei dem die Mischung in Stufe (c) auf eine Teilchengröße von 50 bis 1000 µm zerkleinert wird.

13. Verfahren nach einem der Ansprüche 1 bis 12, bei dem in Stufe (d) die Mischung klassiert wird.

14. Verfahren nach einem der Ansprüche 1 bis 13, bei dem in Stufe (d) die Mischung in Kapseln oder Beutel abgepackt wird.

15. Verfahren nach einem der Ansprüche 1 bis 14, bei dem in Stufe (d) die Mischung, gegebenenfalls nach vorheriger Granulierung, zu Tabletten verarbeitet wird.

16. Pharmazeutische Zusammensetzung, die durch ein Verfahren nach einem der Ansprüche 1 bis 15 erhältlich ist.

17. Pharmazeutische Zusammensetzung nach Anspruch 16, die in Form von Kapseln, Beuteln, Styli, Tabletten oder Minitabletten vorliegt.

18. Pharmazeutische Zusammensetzung nach Anspruch 16 oder 17, die (i) mehr als 25 Gew.-% wirkstoffhaltige Teilchen mit einer Größe von < 2mm in jeder Raumrichtung enthält oder (ii) die bei Kontakt mit wäßrigen Medien zu mehr als 25 Gew.-% zu wirkstoffhaltigen Teilchen mit einer Größe von < 2mm in jeder Raumrichtung zerfällt.

## Claims

1. Process for the preparation of a pharmaceutical composition with sustained drug release, comprising:
(a) providing a mixture which contains a drug and a polymer effecting sustained drug release,
(b) compressing the mixture by passing it between two rollers which have a temperature of more than 40°C and which exert a force in the range from more than 15 and up to 40 kN/cm of roller width on the mixture,
(c) reducing the compressed mixture to the desired particle size, and
(d) optionally further processing of the compressed and reduced mixture.

2. Process according to claim 1, wherein the rollers have a temperature up to 100°C.

3. Process according to claim 1 or 2, wherein the rollers have a temperature in the range of 70 to 90°C.

4. Process according to any one of claims 1 to 3, wherein the rollers exert a force in the range from 18 to 23 kN/cm of roller width on the mixture.

5. Process according to any one of claims 1 to 4, wherein the mixture contains 5 to 90 wt.-% drug.

6. Process according to any one of claims 1 to 5, wherein the mixture contains 2 to 50 wt.-% polymer.

7. Process according to any one of claims 1 to 6, wherein the drug is selected from one of the following groups
alpha- or beta-adrenergics,
alpha- or beta-adrenolytics,
analgesics,
antirheumatics,
antiarthritics,
anticholinergics,
anticonvulsants,
antidepressants,
antiparkinson agents,
antipsychotics,
anxiolytics,
dopamine receptor agonists,
anti-migraine agents,
neuroleptics,
neuroprotectives,
nootropic agents,
non-steroidal antirheumatics,
sedatives and hypnotics.

8. Process according to claim 7, wherein the drug is an anticonvulsant, an antidepressant, an antiparkinson agent, an antipsychotic, an anxiolytic or a dopamine receptor agonist.

9. Process according to claim 8, wherein the drug is oxcarbazepine, valproic acid or a salt thereof, sulthiame, carbamazepine, lamotrigine or levetiracetame.

10. Process according to any one of claims 1 to 9, wherein the polymer is an acrylic acid polymer or acrylic acid copolymer or a salt thereof.

11. Process according to any one of claims 1 to 10, wherein the mixture in step (a) additionally contains at least one substance from the group of colorant, flow regulator, lubricant, dry binder, disintegrant and stabilizer.

12. Process according to any one of claims 1 to 11, wherein the mixture in step (c) is reduced to a particle size of 50 to 1000 µm.

13. Process according to any one of claims 1 to 12, wherein in step (d) the mixture is classified.

14. Process according to any one of claims 1 to 13, wherein in step (d) the mixture is packaged in capsules or pouches.

15. Process according to any one of claims 1 to 14, wherein in step (d) the mixture, optionally after prior granulation, is processed to tablets.

16. Pharmaceutical composition which is obtainable by a process according to any one of claims 1 to 15.

17. Pharmaceutical composition according to claim 16, which is present in the form of capsules, pouches, styli, tablets or minitablets.

18. Pharmaceutical composition according to claim 16 or 17, which contains (i) more than 25 wt.-% particles containing drug and measuring < 2 mm in every direction in space or (ii) which upon contact with aqueous media disintegrates into more than 25 wt.-% particles containing drug and measuring < 2 mm in every direction in space.

## Revendications

1. Procédé de fabrication d'une composition pharmaceutique à libération retardée du principe actif, dans lequel
(a) on fournit un mélange qui contient un principe actif et un polymère induisant une libération retardée du principe actif,
(b) on comprime le mélange en le faisant passer entre deux cylindres qui présentent une température supérieure à 40 °C et qui exercent sur le mélange une force dans la plage de plus de 15 et jusqu'à 40 kN/cm de largeur de cylindre,
(c) le mélange comprimé est broyé à la taille particulaire souhaitée et
(d) éventuellement, le mélange comprimé et broyé est soumis à un traitement subséquent.

2. Procédé selon la revendication 1, dans lequel les cylindres présentent une température allant jusqu'à 100 °C.

3. Procédé selon la revendication 1 ou 2, dans lequel les cylindres présentent une température dans la plage de 70 à 90 °C.

4. Procédé selon l'une quelconque des revendications 1 à 3, dans lequel les cylindres exercent une force dans la plage de 18 à 23 kN/cm de largeur de cylindre.

5. Procédé selon l'une quelconque des revendications 1 à 4, dans lequel le mélange contient 5 à 90 % en poids de principe actif.

6. Procédé selon l'une quelconque des revendications 1 à 5, dans lequel le mélange contient 2 à 50 % en poids de polymère.

7. Procédé selon l'une quelconque des revendications 1 à 6, dans lequel le principe actif est choisi parmi l'un des groupes suivants :
agents alpha- ou bêta-adrénergiques,
agents alpha- ou bêta-adrénolytiques,
agents analgésiques,
agents antirhumatismaux,
agents antiarthritiques,
agents anticholinergiques,
agents anticonvulsifs,
agents antidépresseurs,
agents antiparkinsoniens,
agents antipsychotiques,
agents anxiolytiques,
agonistes des récepteurs de dopamine,
agents antimigraineux,
agents neuroleptiques,
agents neuroprotecteurs,
agents nootropes,
agents antirhumatismaux non stéroïdiens,
agents sédatifs et
agents hypnotiques.

8. Procédé selon la revendication 7, dans lequel le principe actif est un agent anticonvulsif, un agent antidépresseur, un agent antiparkinsonien, un agent antipsychotique, un agent anxiolytique ou un agoniste de récepteurs de dopamine.

9. Procédé selon la revendication 8, dans lequel le principe actif est l'oxcarbazépine, l'acide valproïque ou un de leurs sels, le sultiame, la carbamazépine, la lamotrigine ou le lévetiracétame.

10. Procédé selon l'une quelconque des revendications 1 à 9, dans lequel le polymère est un polymère d'acide acrylique ou un copolymère d'acide acrylique ou un de leurs sels.

11. Procédé selon l'une quelconque des revendications 1 à 10, dans lequel le mélange, à l'étape (a), contient en ou au moins une substance du groupe constitué des colorants, des agents régulateurs d'écoulement, des agents lubrifiants, des liants secs, des adjuvants de désagrégation et des stabilisateurs.

12. Procédé selon l'une quelconque des revendications 1 à 11, dans lequel, à l'étape (c), on broie le mélange à une taille particulaire de 50 à 1000 µm.

13. Procédé selon l'une quelconque des revendications 1 à 12, dans lequel on classe le mélange à l'étape (d).

14. Procédé selon l'une quelconque des revendications 1 à 13, dans lequel on conditionne le mélange en capsules ou en sachets à l'étape (d).

15. Procédé selon l'une quelconque des revendications 1 à 14, dans lequel on transforme le mélange en comprimés à l'étape (d), éventuellement après une granulation antérieure.

16. Composition pharmaceutique susceptible d'être obtenue par un procédé selon l'une quelconque des revendications 1 à 15.

17. Composition pharmaceutique selon la revendication 16, qui se présente sous la forme de capsules, de sachets, de stylos injecteurs, de comprimés ou de minicomprimés.

18. Composition pharmaceutique selon la revendication 16 ou 17, qui (i) contient plus de 25 % en poids de particules contenant un principe actif avec une taille < 2 mm dans chaque direction spatiale ou qui (ii) se décompose, au contact avec des milieux aqueux, à plus de 25 % en poids en particules contenant un principe actif avec une taille < 2 mm dans chaque direction spatiale.
